# EUROPÄISCHE PATENTSCHRIFT

(11) **EP 2 647 359 B1**
(45) Veröffentlichungstag und Bekanntmachung des Hinweises auf die Patenterteilung: **19.03.2014**
(21) Anmeldenummer: 12002401.3
(22) Anmeldetag: 02.04.2012
(51) Int. Cl.: A61F 13/00, A61F 13/64, A61F 15/00, A61B 17/132

(54) **Druckverband**
Compression bandage
Bande compressive

(43) Veröffentlichungstag der Anmeldung: 09.10.2013
(73) Patentinhaber: Karl Werkmeister, Medizinische Leibbinden Inh. Hans-Jürgen Germerodt, 37281 Wanfried (DE)
(72) Erfinder: Ortmann, Jörg, 37269 Eschwege (DE); Schuhmann, Mike, 38642 Goslar (DE)
(74) Vertreter: Walther, Walther & Hinz GbR

(56) Entgegenhaltungen:
- WO-A1-2011/012805
- WO-A2-2011/031004
- US-A- 5 269 803
- US-A- 5 295 996
- US-A1- 2004 092 999

## Beschreibung

Die Erfindung betrifft einen Druckverband für eine Wunde in einem Blut führenden Gefäß, z. B. in einer Arterie in einer Extremität, umfassend ein Kompressorium und eine am Kompressorium angeordnete Bandage, um das Kompressorium auf der Extremität zu fixieren, wobei das Kompressorium an einem Ende die Bandage aufnimmt, wobei das Kompressorium am anderen Ende ein Verschlussmittel zur verstellbaren Fixierung der Bandage aufweist.

Durch die Arterie fließt das Blut vom Herzen zurück in die Extremitäten des Körpers. Das heißt, die Arterie steht unter Überdruck. Dies im Gegensatz zu der Vene, die das Blut zu dem Herzen hin befördert. Zu unterschiedlichen Anlässen wird z. B. die Arterie punktiert. Das heißt, es wird mittels eines z. B. Katheders oder Kanüle ein Zugang in ein Gefäß z. B. eine Arterie gelegt. Wird der Zugang entfernt, mithin z. B. der Katheder aus der Arterie gezogen, so muss, um ein Verschließen der Arterie zu gewährleisten, Druck auf die Punktion in der Arterie ausgeübt werden. Hierzu sind sogenannte Druckverbände bekannt, die über ein Kissen speziell auf die Punktion der Arterie einen gewissen Druck ausüben. Das Kissen kann hierbei aus einem Stück eines Mullverbandes oder Ähnliches gebildet sein.

Aus der US-Schrift 4,005,709 ist ein Druckverband bekannt, wobei dieser Druckverband einen aus einem elastischen Material hergestellten Bandagenabschnitt aufweist, der an seinem einen Ende ein Kissen aufweist, wobei das Kissen einen über dem Kissen angeordneten Druckkörper zeigt, wobei das andere Ende des Bandagenabschnittes ein Verschlussmittel aufweist.

Aus dem Gebrauchsmuster DE 20 2011 101 139 U1 ist ein Kompressorium zum Abdrücken einer Wunde eines Patienten bekannt, das auf der Innenseite eine Aussparung zur Aufnahme des Blutgefäßes aufweist. Dieses Kompressorium wird durch einen Verband an dem Arm des Patienten befestigt.

Aus der WO 2011/012805 A1 ergibt sich ein Druckverband, bei der die Bandage formschlüssig am Kompressorium fixierbar ist. Hierzu besitzt das Kompressorium einen Stift, der durch eine entsprechende Öffnung in der Bandage hindurchführbar ist.

Aus der WO 2011/031004 A2 ist ein Druckverband bekannt, wobei eine Bandage und ein Kompressorium vorgesehen sind, wobei die Bandage mittels eines Klettbandverschlusses verschließbar ist. In gleicher Weise ist der Druckverband gemäß der US 2004/0092999A1 ausgebildet, und zwar insofern, als auch hier die Bandage ebenfalls mittels eines Klettverschlusses verschließbar ist.

Die US 5,269,803 A zeigt einen Druckverband mit einem Kunststoffstreifen, wobei die Bandage nach Art eines Kabelverbinders ausgebildet ist, dass heißt, dass die Fixierung des Kunststoffstreifens durch eine Rasteinrichtung erfolgt. Aus der US 5,295,996 A wiederum ist ein Druckverband bekannt, bei dem die Bandage über einen Klettbandverschluss verfügt.

Aus der EP 2 193 751 B1 ist ein Druckverband der eingangs genannten Art bekannt. Hierbei ist im Einzelnen ein aus einem elastischen Material hergestellter Bandagenabschnitt vorgesehen, der an einem Ende ein Kissen aufweist, wobei das Kissen einen über dem Kissen angeordneten Druckkörper zeigt, wobei das andere Ende des Bandagenabschnittes ein Verschlussmittel besitzt. Der Bandagenabschnitt weist im Bereich des Druckkörpers eine Öse zum Durchziehen des anderen Endes des Bandagenabschnittes mit dem Verschlussmittel auf, wobei die Öse derart an dem Bandagenabschnitt angeordnet ist, dass im geschlossenen Zustand des Verbandes der Bandagenabschnitt über den Druckkörper geführt ist, sodass der Druckkörper unter großem Druck auf der Punktion anliegt.

Dieser Druckverband hat sich äußerst gut bewährt, lediglich die Handhabung hinsichtlich der Fixierung des Bandagenabschnittes unter Last ist etwas aufwendig.

Die der Erfindung zugrunde liegende Aufgabe besteht demzufolge darin, einen Druckverband bereitzustellen, bei dem die Handhabung beim Anlegen verbessert ist.

Zur Lösung der Aufgabe wird bei einem Druckverband der eingangs genannten Art vorgeschlagen, dass das Verschlussmittel ein relativ zum Kompressorium bewegliches Klemmglied zur Fixierung der Bandage an dem Kompressorium durch eine kraftschlüssige Verbindung zwischen Bandage und Kompressorium aufweist. Hieraus wird deutlich, dass durch das Klemmglied die Fixierung der Bandage unmittelbar am Kompressorium erfolgt. Nach dem Stand der Technik gemäß der bereits zuvor erwähnten EP 2 193 751 B1 ist es hingegen so, dass die Fixierung des Druckkörpers auf der Punktion unter Last dadurch erfolgt, dass das Ende des Bandagenabschnittes einen Klett- oder Flauschbandverschluss aufweist, der auf dem Bandagenabschnitt selbst, teilweise im weiten Abstand zu dem Druckkörper, fixiert ist. Der Bandagenabschnitt besteht aus einem elastischen Material. Dies hat zur Folge, dass bereits nach kurzer Zeit aufgrund der Eigenelastizität des Bandagenabschnittes der Druck durch den Druckkörper auf die Punktion in der Arterie nachlässt mit der Folge, dass der Druckverband nachgespannt werden muss. Dies ist nicht nur umständlich und zeitintensiv, sondern kann unter Umständen auch dazu führen, dass bei zu geringer Belastung der Punktion in der Arterie, bereits innerhalb kürzester Zeit relativ große Mengen an Blut austreten können. Insofern ist man bestrebt, den Druckkörper unter verhältnismäßig großem Druck auf die Punktion zu drücken, was allerdings zu venösen Stauungen führen kann.

Durch das erfindungsgemäße Verschlussmittel wird dies insbesondere dadurch vermieden, dass die Bandage unmittelbar am Kompressorium fixiert wird.

Vorteilhafte Merkmale und Ausgestaltungen zu der Erfindung ergeben sich aus den Unteransprüchen.

So ist insbesondere vorgesehen, dass das Klemmglied als Spange ausgebildet ist, wobei das Kompressorium einen Schlitz zur Durchführung der Bandage aufweist, wobei die Spange über den Schlitz verschiebbar ist. Die Spange ist jeweils derart ausgebildet, dass sie in ihrer Lage über dem Schlitz die Bandage klemmend erfasst. Im Einzelnen weist die Spange in diesem Zusammenhang einen Bügel auf, der auf der Führung des Kompressoriums verschieblich gehalten wird. Der Abstand zur Oberseite der Führung des Kompressoriums ist hierbei derart gewählt, dass die Bandage, die aus dem Schlitz hervortritt, durch den Bügel parallel zur Führung gehalten ist, also die Bandage durch den Bügel umgelegt wird. Hierbei entsteht eine kraftschlüssige Verbindung zwischen Bandage und Kompressorium. Es hat sich in diesem Zusammenhang herausgestellt, dass nicht die Gefahr besteht, dass der Verschluss vom Patient unbeabsichtigt geöffnet wird. Dies im Gegensatz zu einem Verschluss, der z. B. als Klettverschluss ausgebildet ist.

Die Führung erstreckt sich zu beiden Seiten des Schlitzes, der der Durchführung der Bandage dient. Hierbei ist die Führung mehr als doppel so breit wie die Spange selbst bzw. der Bügel der Spange. Das bedeutet, dass in einer Stellung der Spange der Schlitz vollständig frei ist, sodass die Bandage durch den Schlitz durchgezogen werden kann, bis das Kompressorium stramm an der Extremität des Patienten anliegt, um dann die Spange so weit über den Schlitz zu verschieben, dass die Bandage zwischen Spange und Führung eingeklemmt ist.

Nach einem weiteren Merkmal der Erfindung weist die Führung einen Anschlag für die Spange auf, sodass die Spange unverlierbar mit dem Kompressorium verbunden ist.

Das Kompressorium besitzt nach einem weiteren Merkmal der Erfindung eine Druckleiste zur Auflage auf der Wunde, d. h. der Punktion. Die Druckleiste ist hierbei Bestandteil des Kompressoriums, d. h., dass das Kompressorium einschließlich der Druckleiste einteilig hergestellt ist, beispielsweise im Kunststoffspritzgussverfahren. Eine Druckleiste aus Kunststoff ist stabiler als ein Kompressorium z. B. in Form eines Polystyrol-Kissens, das nachgiebig ist und insofern der Verband gegebenenfalls nachgespannt werden muss.

Nach einem weiteren vorteilhaften Merkmal zeigt die Druckleiste ein Sichtfenster, um die Punktion sichtbar werden zu lassen. Hiermit soll sichergestellt werden, dass das Kompressorium derart an der Extremität, beispielsweise dem Arm des Patienten, fixiert ist, dass die Druckleiste mit Sicherheit die Punktion erfasst.

Nach einem weiteren Merkmal der Erfindung weist die Druckleiste an zumindest einem Ende eine Ausnehmung oder Kerbe für den in der Ader einsitzenden Zugang auf. Vorteilhaft ist eine derartige Ausnehmung zu beiden Seiten der Druckleiste vorgesehen, sodass der Druckverband sowohl beispielsweise am rechten Arm als auch am linken Arm eingesetzt werden kann.

Das Kompressorium weist einen Rahmen auf, wobei die Druckleiste durch den Rahmen aufgenommen ist, wobei die Druckleiste über dem Rahmen übersteht. Der Überstand der Druckleiste über dem Rahmen ist hierbei derart, dass mit Sicherheit ein genügender Druck auf die beispielsweise Arterie im Bereich der Punktion ausgeübt werden kann, um die Arterie zu verschließen.

Nach einem weiteren Merkmal der Erfindung ist im Bereich der Druckleiste eine Schutzlasche vorgesehen, wobei die Schutzlasche dafür sorgt, dass beim Anlegen des Druckverbandes beim Anziehen der Bandage nicht die Haut des Patienten eingeklemmt wird. Die Schutzlasche ist insofern federnd nachgiebig an der Unterseite des Kompressoriums angeordnet, wobei die Unterseite des Kompressoriums die Seite ist, in dessen Richtung die Druckleiste ausgewölbt ist.

Anhand der Zeichnungen wird die Erfindung nachstehend beispielhaft näher erläutert.
- Fig. 1: zeigt die Anordnung des Druckverbandes im Bereich des Handgelenkes eines Patienten;
- Fig. 2: zeigt den Druckverband in perspektivischer Darstellung;
- Fig. 3: zeigt eine Seitenansicht des Druckverbandes;
- Fig. 4: zeigt eine Ansicht gemäß der Linie IV-IV aus Fig. 2 auf den Druckverband;
- Fig. 5a u. 5b: zeigen einen Schnitt gemäß der Linie V aus Fig. 4, wobei aus den Fig. 5a und 5b der Verschlussmechanismus erkennbar ist;
- Fig. 6: zeigt das Kompressorium in einer perspektivischen Ansicht.

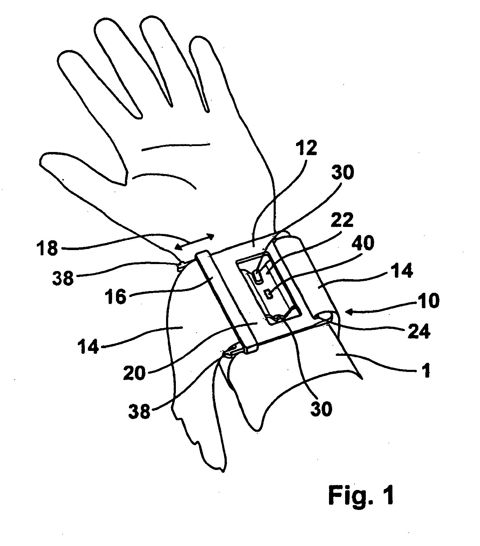

Aus Fig. 1 ist die Anordnung des Druckverbandes am Arm des Patienten erkennbar. Insbesondere ist hierbei erkennbar, dass der Druckverband im Bereich des Handgelenkes (Radialis) angeordnet ist, wobei darauf hingewiesen wird, dass der Druckverband natürlich auch im Bereich der Armbeuge (Brachialis) Verwendung finden kann.

Der Arm des Patienten ist mit 1 bezeichnet. Der insgesamt mit 10 bezeichnete Druckverband besitzt das Kompressorium 12 und die Bandage 14. Die Bandage 14 dient der Fixierung des Kompressoriums 12 auf der Unterseite des Armes 1 des Patienten, wie dies unmittelbar aus Fig. 1 erkennbar ist.

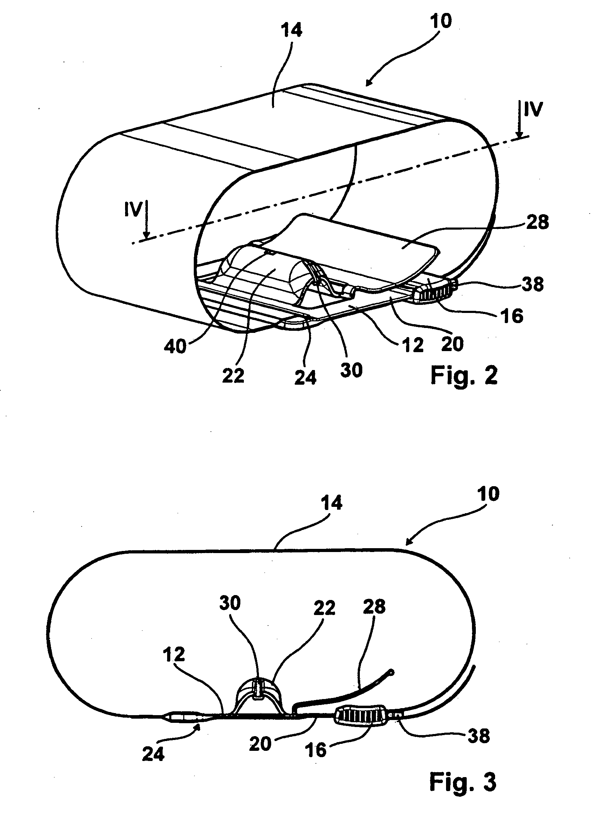

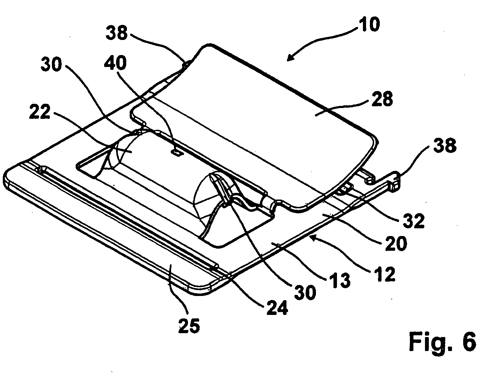

Erkennbar ist darüber hinaus aus Fig. 1 auch die Spange 16, die der Fixierung des offenen Endes der Bandage 14 an dem Kompressorium dient, in dem nämlich die Spange 16 mit dem Bügel 17 in Richtung des Doppelpfeiles 18 auf einer Führung 20 des Kompressoriums 12 verschieblich angeordnet ist. Hierauf wird im Folgenden noch näher eingegangen werden. Erkennbar ist darüber hinaus die Druckleiste 22 des Kompressoriums 12, die unter Druck auf der Punktion der Arterie aufliegt und schlussendlich dazu dient, dass die Arterie verschlossen wird. Das Kompressorium 12 umfasst einen Rahmen 13, wobei durch den Rahmen 13 die Druckleiste 22 mit dem Fenster 40 zur Sichtbarmachung der Punktion aufgenommen ist. Die Druckleiste 22 steht hierbei, wie dies beispielsweise in Anschauung von Fig. 6 ersichtlich ist, weit über den Rahmen des Kompressoriums 12 über, um einen entsprechenden Druck auf die Arterie aufbauen zu können. Darüber hinaus zeigt das Kompressorium 12 einen Schlitz 24 zur Fixierung der Bandage 14. Die Fixierung der Bandage 14 in dem Schlitz 24 erfolgt dadurch, dass durch die Bandage eine Öse gebildet wird, die den Steg 25 zwischen dem Schlitz 24 und der Endkante des Kompressoriums umgibt. Aus den Fig. 2 bis 4 sind die konstruktiven Einzelheiten des Druckverbandes erkennbar. So ist insbesondere aus der perspektivischen Darstellung gemäß Fig. 2 die Schutzlasche 28 erkennbar, die dazu dient, zu verhindern, dass beim Anziehen des Druckverbandes die Haut des Patienten eingeklemmt wird. Erkennbar ist darüber hinaus aus Fig. 2, dass die Druckleiste 22 zu beiden Enden, d. h. parallel zur Blut führenden Ader jeweils eine Ausnehmung 30 zeigt, wobei die Ausnehmung 30 zum einen als Orientierung für die Anlage des Kompressoriums dient, aber darüber hinaus auch als Führung für eine Schleuse fungieren kann, die gegebenenfalls in der Vene oder Arterie des Patienten für eine gewisse Zeit verbleiben soll. Das heißt, dass sich in dieser Ausnehmung oder Kerbe 30 die Schleuse einlegt, und somit durch den Druckverband nicht nur die Punktion einer Arterie verschlossen werden kann, sondern darüber hinaus auch die in der Arterie beispielsweise einliegende Schleuse fixiert wird. Unter einer Schleuse versteht man hierbei ein Kunststoffröhrchen, dessen Ende eine Nadel aufweist, die in die Arterie hineinragt. Des Weiteren zeigt die Druckleiste ein Sichtfenster 40, um die Punktion sehen zu können. Alternativ kann vorgesehen sein, das Kompressorium insgesamt aus einem durchsichtigen Kunststoff z. B. einem ABS herzustellen. Erkennbar ist aus den Fig. 2 bis 4 darüber hinaus wiederum der Schlitz 24, der der Anbindung der Bandage 14 dient.

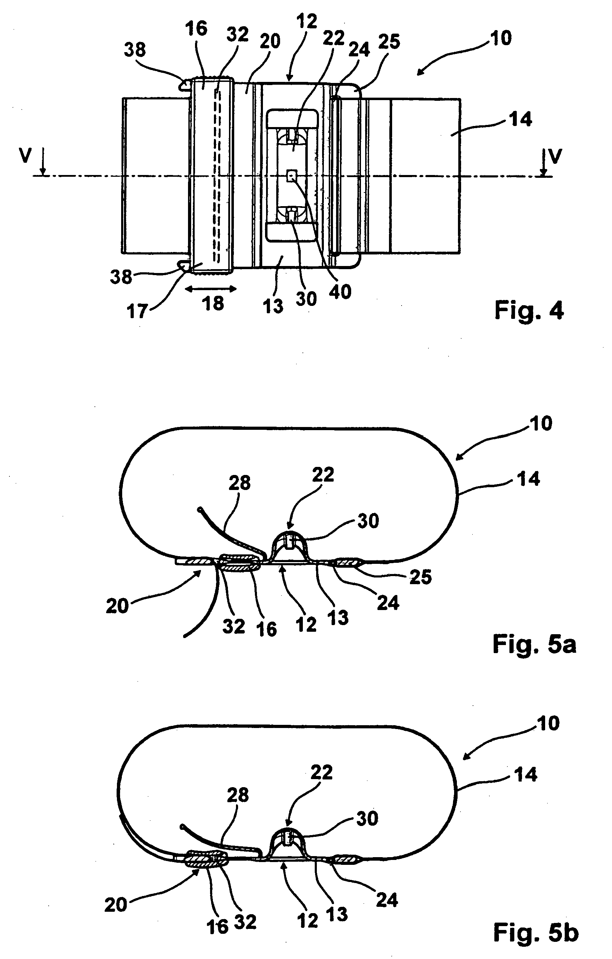

Bei Betrachtung der Fig. 5a und 5b wird erkennbar, wie die Bandage 14 an dem Kompressorium fixiert wird. Die Bandage wird zunächst durch den Schlitz 32 in der Führung 20 des im Kompressoriums 12 geführt, wobei die Bandage so weit angezogen wird, dass die Druckleiste 22 mit dem entsprechenden Druck auf der Punktion der Arterie aufliegt. Die Spange 16 befindet sich hierbei in einer Stellung auf der Führung 20 des Kompressoriums, wie in Fig. 5a dargestellt, nämlich in einer Stellung, bei der der Schlitz 32 völlig freiliegt. Zur Fixierung der Bandage 14 an dem Kompressorium wird nun die Spange in eine Stellung gemäß Fig. 5b überführt, wobei hierbei die Bandage 14 auf die Führung 20 gedrückt gehalten wird. Hierdurch ist die Bandage 14 unverschieblich mit dem Kompressorium verbunden. Um zu verhindern, dass die Spange 16 aus dem Bereich der Führung 20 gelangen kann, besitzt die Führung endseitig einen Anschlag 38. Bei Anliegen der Spange 16 an dem Anschlag 38 überdeckt der Bügel 17 der Spange den Schlitz 32 für die Bandage 14.

### Bezugszeichenliste:

- 1: Arm
- 10: Druckverband
- 12: Kompressorium
- 13: Rahmen des Kompressoriums
- 14: Bandage
- 16: Spange
- 17: Bügel der Spange
- 18: Pfeil
- 20: Führung des Kompressoriums
- 22: Druckleiste
- 24: Schlitz für Bandagenöse
- 25: Steg
- 28: Schutzlasche
- 30: Ausnehmung
- 32: Schlitz für Bandagenende
- 38: Anschlag für Spange
- 40: Sichtfenster

## Patentansprüche

1. Druckverband (10) für eine Wunde in einem Blut führenden Gefäß, z. B. in einer Arterie in einer Extremität, umfassend ein Kompressorium (12) und eine am Kompressorium angeordnete Bandage (14), um das Kompressorium auf der Extremität zu fixieren, wobei das Kompressorium (12) an einem Ende die Bandage (14) aufnimmt und wobei das Kompressorium am anderen Ende ein Verschlussmittel zur verstellbaren Fixierung der Bandage aufweist,
wobei das Verschlussmittel ein relativ zum Kompressorium (12) bewegliches Klemmglied zur Fixierung der Bandage (14) an dem Kompressorium durch eine kraftschlüssige Verbindung zwischen Bandage und Kompressorium aufweist.

2. Druckverband nach Anspruch 1,
**dadurch gekennzeichnet,**
**dass** das Klemmglied als Spange (16) ausgebildet ist, wobei das Kompressorium (12) einen Schlitz (32) zur Durchführung der Bandage (14) aufweist, wobei die Spange (16) über den Schlitz (32) verschiebbar ist.

3. Druckverband nach Anspruch 2,
**dadurch gekennzeichnet,**
**dass** das Kompressorium (12) im Bereich des Schlitzes (32) eine Führung (20) zur verschieblichen Aufnahme der Spange (16) aufweist.

4. Druckverband nach Anspruch 3,
**dadurch gekennzeichnet,**
**dass** die Führung (20) sich zu beiden Seiten des Schlitzes (32) erstreckt.

5. Druckverband nach einem der Ansprüche 3 oder 4,
**dadurch gekennzeichnet,**
**dass** die Führung (20) etwas mehr als doppelt so breit ist wie die Spange (16).

6. Druckverband nach einem der Ansprüche 3 bis 5,
**dadurch gekennzeichnet,**
**dass** die Führung (20) einen Anschlag (38) für die Spange (16) aufweist.

7. Druckverband nach einem der Ansprüche 2 bis 6,
**dadurch gekennzeichnet,**
**dass** die Spange (16) einen Bügel (17) aufweist, der einen Abstand zu der Oberseite der Führung (20) zeigt.

8. Druckverband nach einem der vorangehenden Ansprüche,
**dadurch gekennzeichnet,**
**dass** das Kompressorium (12) eine Druckleiste (22) zur Auflage auf der Wunde aufweist.

9. Druckverband nach Anspruch 8,
**dadurch gekennzeichnet,**
**dass** die Druckleiste (22) ein Sichtfenster (40) aufweist.

10. Druckverband nach einem der Ansprüche 8 oder 9 ,
**dadurch gekennzeichnet,**
**dass** die Druckleiste (22) an zu mindest einem Ende eine Ausnehmung (30) für eine in der Ader einsitzende Schleuse aufweist.

11. Druckverband nach einem der voranstehenden Ansprüche,
**dadurch gekennzeichnet,**
**dass** das Kompressorium (12) einen Rahmen (13) aufweist, wobei eine Druckleiste (22) durch den Rahmen (13) aufgenommen ist, wobei die Druckleiste (22) über den Rahmen (13) übersteht.

12. Druckverband nach einem der Ansprüche 8 - 11,
**dadurch gekennzeichnet,**
**dass** das Kompressorium (12) im Bereich der Druckleiste (22) eine Schutzlasche (28) aufweist.

13. Druckverband nach Anspruch 12,
**dadurch gekennzeichnet,**
**dass** die Schutzlasche (28) federnd nachgiebig an der Unterseite des Kompressoriums (12) angeordnet ist.

14. Druckverband nach einem der vorangehenden Ansprüche,
**dadurch gekennzeichnet,**
**dass** das Kompressorium (12) als Kunststoffspritzteil ausgebildet ist.

## Claims

1. A compression bandage (10) for a wound in a blood vessel, e.g. in an artery of an extremity, including a compressorium (12) and a bandage (14) disposed on the compressorium for fixing the compressorium onto the extremity, the compressorium (12) accommodating the bandage (14) at one end and the compressorium comprising a closing means at its other end for adjustably fixing the bandage,
the closing means comprising a clamping member, which is movable relative to the compressorium (12) for fixing the bandage (14) on the compressorium by way of a frictional connection between the bandage and the compressorium.

2. The compression bandage according to claim 1,
**characterized in that**
the clamping member is configured as a clasp (16), the compressorium (12) comprising a slot (32) through which the bandage (14) is passed, the clasp (16) being displaceable above the slot (32).

3. The compression bandage according to claim 2,
**characterized in that**
the compressorium (12) comprises a guide (20) in the area of the slot (32) for displaceably receiving the clasp (16).

4. The compression bandage according to claim 3,
**characterized in that**
the guide (20) extends on both sides of the slot (32).

5. The compression bandage according to one of the claims 3 or 4,
**characterized in that**
the guide (20) is a little more than twice as wide as the clasp (16).

6. The compression bandage according to one of the claims 3 to 5,
**characterized in that**
the guide (20) comprises a limit-stop (38) for the clasp (16).

7. The compression bandage according to one of the claims 2 to 6,
**characterized in that**
the clasp (16) comprises a bow (17) at a distance from the upper side of the guide (20).

8. The compression bandage according to one of the afore-mentioned claims,
**characterized in that**
the compressorium (12) comprises a pressure strip (22) for resting on the wound.

9. The compression bandage according to claim 8,
**characterized in that**
the pressure strip (22) comprises a viewing window (40).

10. The compression bandage according to one of the claims 8 or 9,
**characterized in that**
the pressure strip (22) comprises a recess (30) at at least one end for a sheath resting in the artery.

11. The compression bandage according to one of the afore-mentioned claims,
**characterized in that**
the compressorium (12) comprises a frame (13), a pressure strip (22) being accommodated in the frame (13), the pressure strip (22) protruding from the frame (13).

12. The compression bandage according to one of the claims 8 - 11,
**characterized in that**
the compressorium (12) comprises a protective flap (28) in the area of the pressure strip (22).

13. The compression bandage according to claim 12,
**characterized in that**
the protective flap (28) is disposed on the underside of the compressorium (12) in an elastically flexible manner.

14. The compression bandage according to one of the afore-mentioned claims,
**characterized in that**
the compressorium (12) is configured as an injection-molded plastic part.

## Revendications

1. Pansement de compression (10) pour une plaie au niveau d'un conduit sanguin, par exemple une artère dans un organe d'extrémité, comportant un dispositif de compression (12) et un bandage (14) associé au dispositif de compression pour fixer le dispositif de compression sur l'organe d'extrémité, dans lequel le dispositif de compression (12) fixe le bandage (14) à une de ses extrémités et dans lequel il comporte à son autre extrémité un moyen de verrouillage en vue de la fixation réglable du bandage,
dans lequel le moyen de verrouillage comprend un organe de pincement, mobile par rapport au dispositif de compression (12), pour la fixation du bandage (14) au dispositif de compression par une liaison de verrouillage forcé entre le bandage et le dispositif de compression.

2. Pansement de compression selon la revendication 1,
**caractérisé en ce que**,
l'organe de pincement est en forme d'agrafe (16), dans lequel le dispositif de compression (12) comprend une fente (32) pour le passage du bandage (14), dans lequel l'agrafe (16) est agencée pour se fermer pardessus la fente (32).

3. Pansement de compression selon la revendication 2,
**caractérisé en ce que**,
le dispositif de compression (12) comporte un guidage (20) dans le secteur de la fente (32) pour la fixation coulissante de l'agrafe (16).

4. Pansement de compression selon la revendication 3,
**caractérisé en ce que**,
le guidage (20) s'étend des deux côtés de la fente (32).

5. Pansement de compression selon l'une des revendications 3 ou 4,
**caractérisé en ce que**,
le guidage (20) est environ deux fois plus large que l'agrafe (16).

6. Pansement de compression selon l'une des revendications 3 à 5,
**caractérisé en ce que**,
le guidage (20) comporte une butée (38) pour l'agrafe (16)

7. Pansement de compression selon l'une des revendications 2 à 6,
**caractérisé en ce que**,
l'agrafe (16) comporte un arceau (17) qui présente un écartement par rapport au côté supérieur du guidage (20).

8. Pansement de compression selon l'une des revendications précédentes,
**caractérisé en ce que**,
le dispositif de compression (12) comporte une liste de pression (22) pour l'appui sur la plaie.

9. Pansement de compression selon la revendication 8,
**caractérisé en ce que**,
la liste de pression (22) comporte une fenêtre d'observation (40).

10. Pansement de compression selon la revendication 8 ou 9,
**caractérisé en ce que**,
la liste de pression (22) comporte à au moins une extrémité, une ouverture (30) pour l'implantation d'une écluse dans l'artère.

11. Pansement de compression selon l'une des revendications précédentes,
**caractérisé en ce que**,
le dispositif de compression (12) comporte un cadre (13), dans lequel une liste de pression (22) est maintenue par le cadre (13), dans lequel la liste de pression (22) surpasse le cadre (13).

12. Pansement de compression selon l'une des revendications 8 - 11,
**caractérisé en ce que**,
le dispositif de compression (12) comporte une languette de protection (28) dans le secteur de la liste de pression (22).

13. Pansement de compression selon la revendication 12,
**caractérisé en ce que**,
la languette de protection (28) est montée à la partie inférieure du dispositif de compression (12) et est flexible en absorbant la contrainte.

14. Pansement de compression selon l'une des revendications précédentes,
**caractérisé en ce que**,
le dispositif de compression (12) est réalisé sous la forme d'une pièce injectée en matière synthétique.
